# EUROPEAN PATENT SPECIFICATION

(11) **EP 3 976 596 B1**
(45) Date of publication and mention of the grant of the patent: **19.07.2023**
(21) Application number: 20862006.2
(22) Date of filing: 11.11.2020
(51) Int. Cl.: C07D 401/04

(54) **METHODS FOR THE PREPARATION OF ETHYL 3-BROMO-1-(3-CHLOROPYRIDIN-2-YL)-1H-PYRAZOLE-5-CARBOXYLATE**
VERFAHREN ZUR HERSTELLUNG VON 3-BROM-1-(3-CHLORPYRIDIN-2-YL)-1H-PYRAZOL-5-ETHYLCARBONSÄUREETHYLESTER
PROCÉDÉS DE PRÉPARATION DE 3-BROMO-1-(3-CHLOROPYRIDINE-2-YL)-1H-PYRAZOLE-5-CARBOXYLATE D'ÉTHYLE

(30) Priority: 11.11.2019 US 201962933553 P
(43) Date of publication of application: 06.04.2022
(73) Proprietor: FMC Corporation, Philadelphia, Pennsylvania 19104 (US); FMC Agro Singapore Pte. Ltd., Singapore 018983 (SG)
(72) Inventor: JI, Shuren, Philadelphia, PA 19104 (US); LUAN, Jie, Philadelphia, PA 19104 (US); MAO, Jianhua, Philadelphia, PA 19104 (US); WANG, Hao, Philadelphia, PA 19104 (US); XU, Yihui, Philadelphia, PA 19104 (US)
(74) Representative: Dehns
(86) International application number: PCT/US2020/059923
(87) International publication number: WO 2021/096903

(56) References cited:
- WO-A1-03/016283
- WO-A1-2006/102025

## Description

### FIELD OF INVENTION

This disclosure is directed to methods of synthesizing compounds of Formula II including ethyl 3-bromo-1-(3-chloropyridin-2-yl)-1H-pyrazole-5-carboxylate. Compounds prepared by the methods disclosed herein are useful for preparation of certain anthranilamide compounds that are of interest as insecticides, such as, for example, the insecticides chlorantraniliprole and cyantraniliprole.

### BACKGROUND

Ethyl 3-bromo-1-(3-chloropyridin-2-yl)-1H-pyrazole-5-carboxylate is an intermediate in the production of 3-bromo-N-[4-chloro-2-methyl-6-[(methylamino)carbonyl]phenyl]-1-(3-chloro-2-pyridinyl)-1H-pyrazole-5-carboxamide and 3-Bromo-1-(3-chloropyridin-2-yl)-N-[4-cyano-2-methyl-6-[N-methylcarbamoyl]phenyl]-1H-pyrazole-5-carboxamide.

As disclosed in the patent WO03016283A1, 3-bromo-1-(3-chloropyridin-2-yl)-1H-pyrazole-5-carboxylate is produced from ethyl 3-bromo-1-(3-chloropyridin-2-yl)-4,5-dihydro-1H-pyrazole-5-carboxylate through an oxidation reaction in acetonitrile system in the presence of potassium persulfate using sulfuric acid as catalyst. The reported yield is about 75-80%.

WO2006/102025A1 discloses methods of converting 2-pyrazilines to pyrazoles using bromine. The examples of WO2006/102025A1 disclose bromination of ethyl 3 -bromo-1-(3 -chloro-2-pyridinyl)-4, 5-dihydro-1*H*-pyrazole-5-carboxylate.

The present disclosure provides methods useful for preparing compounds such as ethyl 3-bromo-1-(3-chloropyridin-2-yl)-1H-pyrazole-5-carboxylate and derivatives thereof. The benefits of the methods of the present disclosure compared to previous methods are numerous and include improved overall yield, reduced cost, and reduced process hazards.

The disclosed methods provide an overall yield of about 88%.

### BRIEF DESCRIPTION

In one aspect, provided herein is a method of preparing a compound of Formula II, wherein 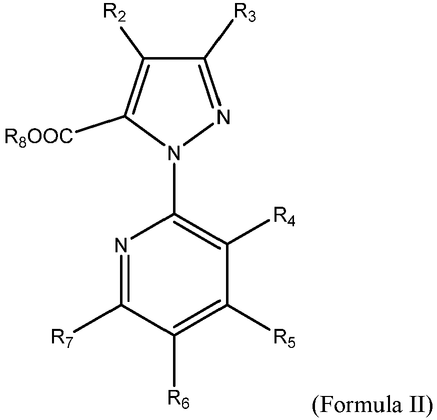
R₈ is selected from hydrogen and C₁-C₄ alkyl; and
each of R₂ - R₇ is independently selected from hydrogen, halogen, C₁-C₄ alkyl, and halogenated C₁-C₄ alkyl, the method comprising
   I) forming a mixture comprising
      A) a first amount of a compound of Formula I, wherein 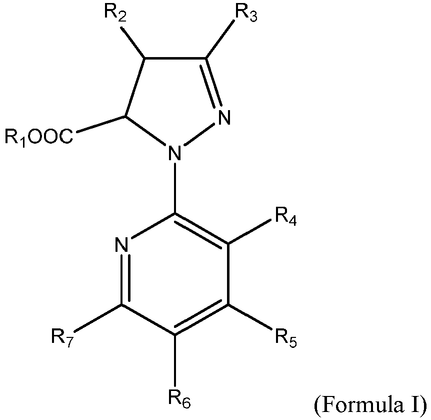
         R₁ is selected from hydrogen and C₁-C₄ alkyl; and
         each of R₂ - R₇ is independently selected from hydrogen, halogen, C₁-C₄ alkyl, and halogenated C₁-C₄ alkyl;
      B) an oxidizing agent; and
      C) an organic solvent;
   II) heating the mixture;
   III) adding an acid to the mixture;
   IV) adding a second amount of the compound of Formula I to the mixture; and
   V) completing the reaction of the mixture.

### DETAILED DESCRIPTION OF THE DISCLOSURE

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where an invention or a portion thereof is defined with an openended term such as "comprising," it should be readily understood that (unless otherwise stated) the description should be interpreted to also describe such an invention using the terms "consisting essentially of" or "consisting of."

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances (i.e. occurrences) of the element or component. Therefore "a" or "an" should be read to include one or at least one, and the singular word form of the element or component also includes the plural unless the number is obviously meant to be singular.

As used herein, the term "about" means plus or minus 10% of the value.

The term "eq" refers to the amount of a substance that reacts with (or is equivalent to) an arbitrary amount of another substance in a given chemical reaction.

The term "% assay" refers to the content of the desired compound divided by the total weight of the sample.

The term "halogen", either alone or in compound words or phrases such as "haloalkyl" or "halogenated alkyl", includes fluorine, chlorine, bromine or iodine. Further, when used in compound words or phrases such as "haloalkyl" or "halogenated alkyl", said alkyl may be partially or fully substituted with halogen atoms which may be the same or different.

When a group contains a substituent which can be hydrogen, for example R⁴, then, when this substituent is taken as hydrogen, it is recognized that this is equivalent to said group being unsubstituted.

Certain compounds prepared in this invention can exist as one or more stereoisomers. The various stereoisomers include enantiomers, diastereomers, atropisomers and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

The embodiments of this disclosure include:
Embodiment 1. A method of preparing a compound of Formula II, wherein 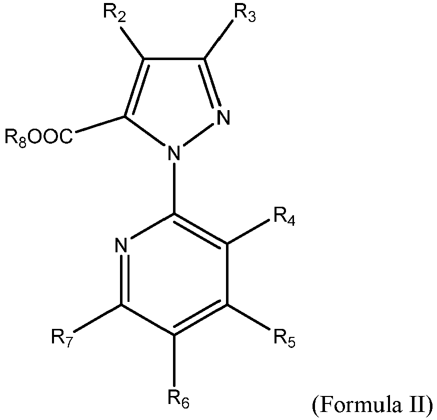
   R₈ is selected from hydrogen and C₁-C₄ alkyl; and
   each of R₂ - R₇ is independently selected from hydrogen, halogen, C₁-C₄ alkyl, and halogenated C₁-C₄ alkyl, the method comprising
      I) forming a mixture comprising
         A) a first amount of a compound of Formula I, wherein 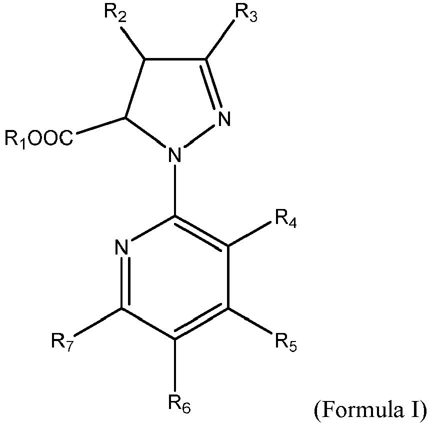
            R₁ is selected from hydrogen and C₁-C₄ alkyl; and
            each of R₂ - R₇ is independently selected from hydrogen, halogen, C₁-C₄ alkyl, and halogenated C₁-C₄ alkyl;
         B) an oxidizing agent; and
         C) an organic solvent;
      II) heating the mixture;
      III) adding an acid to the mixture;
      IV) adding a second amount of the compound of Formula I to the mixture; and
      V) completing the reaction of the mixture.
Embodiment 2. The method of embodiment 1, wherein the oxidizing agent is selected from hydrogen peroxide, organic peroxides, potassium persulfate, sodium persulfate, ammonium persulfate, potassium monopersulfate, sodium monopersulfate, potassium permanganate, and combinations thereof.
Embodiment 3. The method of embodiment 1, wherein the oxidizing agent is potassium persulfate.
Embodiment 4. The method of embodiment 1, wherein the oxidizing agent is present in the mixture in an amount in the range of about 1.0 eq to about 2.0 eq.
Embodiment 5. The method of embodiment 1, wherein the oxidizing agent is present in the mixture in an amount in the range of about 1.3 eq to about 1.7 eq.
Embodiment 6. The method of embodiment 1, wherein the oxidizing agent has a D50 particle size in the range of about 10 µm to about 200 µm.
Embodiment 7. The method of embodiment 1, wherein the oxidizing agent has a D50 particle size in the range of about 20 µm to about 100 µm.
Embodiment 8. The method of embodiment 1, wherein the oxidizing agent has a D50 particle size in the range of about 30 µm to about 80 µm.
Embodiment 9. The method of embodiment 1, wherein the oxidizing agent has a D50 particle size in the range of about 40 µm to about 60 µm.
Embodiment 10. The method of embodiment 1, wherein the solvent is selected from ethers, esters, aprotic organic solvents, and combinations thereof.
Embodiment 11. The method of embodiment 10, wherein the ether is selected from tetrahydrofuran, 2-methyltetrahydrofuran, dioxane, and combinations thereof.
Embodiment 12. The method of embodiment 10, wherein the ester is selected from ethyl acetate, isopropyl acetate, dimethyl carbonate, butyl acetate, and combinations thereof.
Embodiment 13. The method of embodiment 10, wherein the aprotic organic solvent is selected from N,N-dimethylformamide, dimethylacetamide, dimethyl sulfoxide, 1,3-dimethyl-2-imidazolidinone, acetonitrile, and combinations thereof.
Embodiment 14. The method of embodiment 10, wherein the aprotic organic solvent is acetonitrile.
Embodiment 15. The method of embodiment 1, wherein the first amount of the compound of Formula I is about 5 wt% to about 40 wt% of the total amount of the compound of Formula I.
Embodiment 16. The method of embodiment 1, wherein the first amount of the compound of Formula I is present in the mixture in a concentration in the range of about 10 wt% to about 30 wt% of the total amount of the compound of Formula I.
Embodiment 17. The method of embodiment 1, wherein the acid is selected from inorganic acids, organic acids, and combinations thereof.
Embodiment 18. The method of embodiment 17, wherein the organic acid is selected from acetic acid, propanoic acid, p-toluenesulfonic acid, benzoic acid, and combinations thereof.
Embodiment 19. The method of embodiment 17, wherein the inorganic acid is selected from sulfuric acid, phosphoric acid, oleum, hydrobromic acid, hydrochloric acid, and combinations thereof.
Embodiment 20. The method of embodiment 17, wherein the inorganic acid is sulfuric acid.
Embodiment 21. The method of embodiment 1, wherein the acid is present in the mixture in an amount in the range of about 0.05 eq to about 1.5 eq.
Embodiment 22. The method of embodiment 1, wherein the acid is present in the mixture in an amount less than about 0.2 eq.
Embodiment 23. The method of embodiment 1, wherein the method step of forming the mixture occurs at a temperature in the range of about 0 °C to about 60 °C.
Embodiment 24. The method of embodiment 1, wherein the method step of forming the mixture occurs at a temperature in the range of about 15 °C to about 35 °C.
Embodiment 25. The method of embodiment 1, wherein the method step of forming the mixture occurs at room temperature.
Embodiment 26. The method of embodiment 1, wherein the method step of heating the mixture increases the temperature of the mixture to a temperature in the range of about 50 °C to about 82 °C.
Embodiment 27. The method of embodiment 1, wherein the method step of heating the mixture increases the temperature of the mixture to a temperature in the range of about 55 °C to about 65 °C.
Embodiment 28. The method of embodiment 1, wherein the method step of adding a second amount of the compound of Formula I to the mixture occurs at a temperature in the range of about 50 °C to about 82 °C.
Embodiment 29. The method of embodiment 1, wherein the method step of adding a second amount of the compound of Formula I to the mixture occurs at a temperature in the range of about 65 °C to about 82 °C.
Embodiment 30. The method of embodiment 1, wherein the method step of adding a second amount of the compound of Formula I comprises discretely adding the second amount of the compound of Formula I.
Embodiment 31. The method of embodiment 1, wherein the method step of adding a second amount of the compound of Formula I comprises continuously adding the second amount of the compound of Formula I.
Embodiment 32. The method of embodiment 1, wherein the method step of adding a second amount of the compound of Formula I comprises dropwise addition of the second amount of the compound of Formula I.
Embodiment 33. The method of embodiment 1, wherein the method step of adding a second amount of the compound of Formula I occurs over a period of time in the range of about 3 hours to about 7 hours.
Embodiment 34. The method of embodiment 1, wherein the method step of adding a second amount of the compound of Formula I occurs over a period of time in the range of about 3.5 hours to about 4.5 hours.
Embodiment 35. The method of embodiment 1, wherein the second amount of the compound of Formula I is greater than the first amount of the compound of Formula I.
Embodiment 36. The method of embodiment 1, wherein the second amount of the compound of Formula I is at least twice the first amount of the compound of Formula I.
Embodiment 37. The method of embodiment 1, wherein the second amount of the compound of Formula I is about 70 wt% of the total amount of the compound of Formula I.
Embodiment 38. The method of embodiment 1, wherein the first amount of the compound of Formula I is about 30 wt% of the total amount of the compound of Formula I.
Embodiment 39. The method of embodiment 1, wherein at least one method step further comprises detecting the O₂ content of the mixture with an oxygen sensor.
Embodiment 40. The method of embodiment 1, wherein the method step of heating the mixture occurs in the absence of O₂.
Embodiment 41. The method of embodiment 1, wherein the method produces less than about 0.5 wt% O₂.
Embodiment 42. The method of embodiment 1, wherein the method does not produce O₂.
Embodiment 43. The method of embodiment 1, wherein at least one method step further comprises stirring the mixture.

In one aspect, ethyl 3-bromo-1-(3-chloropyridin-2-yl)-1H-pyrazole-5-carboxylate is prepared according to a method represented by Scheme 1.

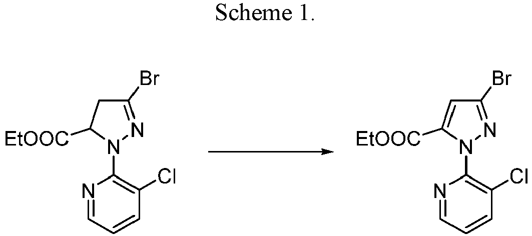

In one aspect, a compound of Formula II is prepared according to a method represented by Scheme 2. The R groups are as defined anywhere in this disclosure.

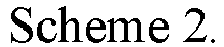

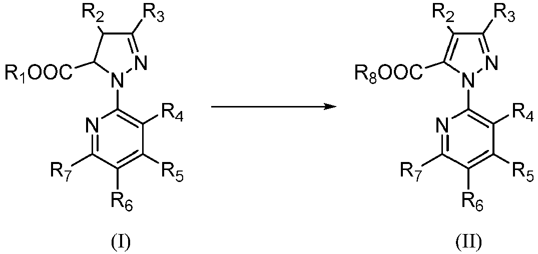

This aspect includes forming a mixture comprising a first amount of a compound of Formula I, an oxidizing agent, and an organic solvent, heating the mixture, adding an acid to the mixture, adding a second amount of the compound of Formula I to the mixture, and completing the reaction of the mixture.

In one embodiment, the oxidizing agent is selected from hydrogen peroxide, organic peroxides, potassium persulfate, sodium persulfate, ammonium persulfate, potassium monopersulfate, sodium monopersulfate, potassium permanganate, and combinations thereof. In another embodiment, the oxidizing agent is potassium persulfate. In one embodiment, the oxidizing agent is present in the mixture in an amount in the range of about 1.0 eq to about 2.0 eq. In another embodiment, the oxidizing agent is present in the mixture in an amount in the range of about 1.3 eq to about 1.7 eq. In one embodiment, the oxidizing agent has a D50 particle size in the range of about 10 µm to about 200 µm. In another embodiment, the oxidizing agent has a D50 particle size in the range of about 20 µm to about 100 µm. In another embodiment, the oxidizing agent has a D50 particle size in the range of about 30 µm to about 80 µm. In another embodiment, the oxidizing agent has a D50 particle size in the range of about 40 µm to about 60 µm.

In one embodiment, the solvent is selected from ethers, esters, aprotic organic solvents, and combinations thereof. In another embodiment, the solvent is an ether selected from tetrahydrofuran, 2-methyltetrahydrofuran, dioxane, and combinations thereof. In another embodiment, the solvent is an ester selected from ethyl acetate, isopropyl acetate, dimethyl carbonate, butyl acetate, and combinations thereof. In one embodiment, the solvent is an aprotic organic solvent selected from N,N-dimethylformamide, dimethylacetamide, dimethyl sulfoxide, 1,3-dimethyl-2-imidazolidinone, acetonitrile, and combinations thereof. In another embodiment, the solvent is acetonitrile.

In one embodiment, the first amount of the compound of Formula I is about 5 wt% to about 40 wt% of the total amount of the compound of Formula I. In another embodiment, the first amount of the compound of Formula I is about 10 wt% to about 30 wt% of the total amount of the compound of Formula I.

In one embodiment, the acid is selected from inorganic acids, organic acids, and combinations thereof. In another embodiment, the acid is an organic acid selected from acetic acid, propanoic acid, p-toluenesulfonic acid, benzoic acid, and combinations thereof. In another embodiment, the acid is an inorganic acid selected from sulfuric acid, phosphoric acid, oleum, hydrobromic acid, hydrochloric acid, and combinations thereof. In another embodiment, the acid is sulfuric acid. In one embodiment, the acid is present in the mixture in an amount in the range of about 0.05 eq to about 1.5 eq. In another embodiment, the acid is present in the mixture in an amount less than about 0.2 eq.

In one embodiment, the method step of forming the mixture occurs at a temperature in the range of about 0 °C to about 60 °C. In another embodiment, the method step of forming the mixture occurs at a temperature in the range of about 15 °C to about 35 °C. In another embodiment, the method step of forming the mixture occurs at room temperature. In one embodiment, the method step of forming the mixture comprises stirring the mixture.

In one embodiment, the method step of heating the mixture increases the temperature of the mixture to a temperature in the range of about 50 °C to about 82 °C. In another embodiment, the method step of heating the mixture increases the temperature of the mixture to a temperature in the range of about 55 °C to about 65 °C. In one embodiment, the method step of heating the mixture comprises stirring the mixture.

In one embodiment, the method step of adding a second amount of the compound of Formula I to the mixture occurs at a temperature in the range of about 50 °C to about 82 °C. In another embodiment, the method step of adding a second amount of the compound of Formula I to the mixture occurs at a temperature in the range of about 50 °C to about 82 °C. In one embodiment, the method step of adding a second amount of the compound of Formula I comprises discretely adding the second amount of the compound of Formula I. In one embodiment, the method step of adding a second amount of the compound of Formula I comprises continuously adding the second amount of the compound of Formula I. In another embodiment, the method step of adding a second amount of the compound of Formula I comprises dropwise addition of the second amount of the compound of Formula I. In one embodiment, the method step of adding a second amount of the compound of Formula I occurs over a period of time in the range of about 3 hours to about 7 hours. In another embodiment, the method step of adding a second amount of the compound of Formula I occurs over a period of time in the range of about 3.5 hours to about 4.5 hours. In one embodiment, the method step of adding a second amount of the compound of Formula I comprises stirring the mixture.

In one embodiment, the second amount of the compound of Formula I is greater than the first amount of the compound of Formula I. In another embodiment, the second amount of the compound of Formula I is at least twice the first amount of the compound of Formula I. In one embodiment, the second amount of the compound of Formula I is about 70 wt% of the total amount of the compound of Formula I. In another embodiment, the first amount of the compound of Formula I is about 30 wt% of the total amount of the compound of Formula I. In one embodiment, the second amount of the compound of Formula I is about 80 wt% of the total amount of the compound of Formula I. In another embodiment, the first amount of the compound of Formula I is about 20 wt% of the total amount of the compound of Formula I.In one embodiment, the second amount of the compound of Formula I is about 90 wt% of the total amount of the compound of Formula I. In another embodiment, the first amount of the compound of Formula I is about 10 wt% of the total amount of the compound of Formula I. In one embodiment, the second amount of the compound of Formula I is about 95 wt% of the total amount of the compound of Formula I. In another embodiment, the first amount of the compound of Formula I is about 5 wt% of the total amount of the compound of Formula I.

In one embodiment, at least one method step further comprises detecting the O₂ content of the mixture with an oxygen sensor. In one embodiment, the method step of heating the mixture occurs in the absence of O₂. In one embodiment, the method produces less than about 0.5 wt% O₂. In another embodiment, the method does not produce O₂. Low and/or nondetectable O₂ production increases process safety.

### EXAMPLES

Without further elaboration, it is believed that one skilled in the art using the preceding description can utilize the present invention to its fullest extent. The following Examples are, therefore, to be construed as merely illustrative. The starting material for the following Examples may not have necessarily been prepared by a particular preparative run whose procedure is described in other Examples. It also is understood that any numerical range recited herein includes all values from the lower value to the upper value. For example, if a range is stated as 10-50, it is intended that values such as 12-30, 20-40, or 30-50, are expressly enumerated in this specification. These are only examples of what is specifically intended, and all possible combinations of numerical values between and including the lowest value and the highest value enumerated are to be considered to be expressly stated in this application.

Comparative Example 1. Method of monitoring oxygen generation.

153 g potassium persulfate, 450 g acetonitrile, and other starting materials are added to a 2L flask equipped with a condenser, thermometer, dropping funnel, and nitrogen input pipe. An oxygen sensor is set up over the condenser. The air in the reactor is replaced with nitrogen flowing at a rate of 0.55 L/min. When the oxygen sensor shows 0.0% oxygen level, the mixture is heated to 60 °C. Then 4.0 g sulfuric acid is added to the mixture and the mixture is kept heating to reflux. During the whole process, the oxygen level is monitored and recorded.

Comparative Example 2. Single addition of ethyl 3-bromo-1-(3-chloropyridin-2-yl)-4,5-dihydro-1H-pyrazole-5-carboxylate at the start of the reaction.

174 g potassium persulfate (1.6eq), 780 g 17% ethyl 3-bromo-1-(3-chloropyridin-2-yl)-4,5-dihydro-1H-pyrazole-5-carboxylate in acetonitrile, and 40 g of concentrated sulfuric acid (1.0 eq) are added to a reactor. The reaction mixture is stirred and heated to 65 °C. The reaction shows obvious exothermic release and thus self-heats to reflux. The reaction mixture is kept at reflux for another 3-4 hours to complete the reaction. No oxygen is detected throughout the process. 350 g water is added to the mixture at 70 °C and the phase is split with potassium bisulfate to remove waste water. After stripping solvent and subsequent crystallization, about 107 g ethyl 3-bromo-1-(3-chloropyridin-2-yl)-1H-pyrazole-5-carboxylate dry product is obtained with about 96% assay and about 78% yield.

Example 1. Addition of ethyl 3-bromo-1-(3-chloropyridin-2-yl)-4,5-dihydro-1H-pyrazole-5-carboxylate at the start of the reaction and after the start of the reaction.

100 g 40% ethyl 3-bromo-1-(3-chloropyridin-2-yl)-4,5-dihydro-1H-pyrazole-5-carboxylate in acetonitrile, 153 g potassium persulfate (1.4 eq), and 450 g acetonitrile are added to a reactor. The reaction mixture is stirred and heated to 60 °C and then 4.0 g concentrated sulfuric acid (0.1 eq) is added to the mixture. The reaction mixture is kept at reflux for 1 hour and then 230 g 40% ethyl 3-bromo-1-(3-chloropyridin-2-yl)-4,5-dihydro-1H-pyrazole-5-carboxylate in acetonitrile is added dropwise during a period of 3-5 hours. After all materials are added, the reaction mixture is kept at reflux for another 1-2 hours to complete the reaction. No oxygen is detected throughout the process. 350g water is added to the mixture at 70 °C and the phase is split with potassium bisulfate to remove waste water. After stripping solvent and subsequent crystallization, about 120 g ethyl 3-bromo-1-(3-chloropyridin-2-yl)-1H-pyrazole-5-carboxylate dry product is obtained with about 97% assay and about 88% yield.

This written description uses examples to illustrate the present disclosure, including the best mode, and also to enable any person skilled in the art to practice the disclosure, including making and using any devices or systems and performing any methods

## Claims

1. A method of preparing a compound of Formula II, wherein 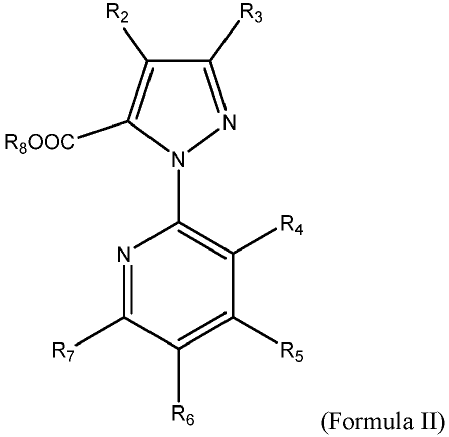
R₈ is selected from hydrogen and C₁-C₄ alkyl; and
each of R₂ - R₇ is independently selected from hydrogen, halogen, C₁-C₄ alkyl, and halogenated C₁-C₄ alkyl, the method comprising
I) forming a mixture comprising
A) a first amount of a compound of Formula I, wherein 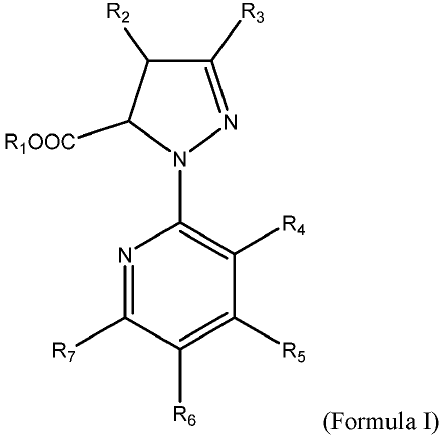
R₁ is selected from hydrogen and C₁-C₄ alkyl; and
each of R₂ - R₇ is independently selected from hydrogen, halogen, Ci-C₄ alkyl, and halogenated C₁-C₄ alkyl;
B) an oxidizing agent; and
C) an organic solvent;
II) heating the mixture;
III) adding an acid to the mixture;
IV) adding a second amount of the compound of Formula I to the mixture; and
V) completing the reaction of the mixture.

2. The method of claim 1, wherein the oxidizing agent is selected from hydrogen peroxide, organic peroxides, potassium persulfate, sodium persulfate, ammonium persulfate, potassium monopersulfate, sodium monopersulfate, potassium permanganate, and combinations thereof.

3. The method of claim 1, wherein the oxidizing agent is present in the mixture in an amount in the range of about 1.0 eq to about 2.0 eq.

4. The method of claim 1, wherein the oxidizing agent has a D50 particle size in the range of about 10 µm to about 200 µm.

5. The method of claim 1, wherein the solvent is selected from ethers, esters, aprotic organic solvents, and combinations thereof.

6. The method of claim 1, wherein the first amount of the compound of Formula I about 5 wt% to about 40 wt% of the total amount of the compound of Formula I, or wherein the first amount of the compound of Formula I is less than 30 wt% of the total amount of the compound of Formula I.

7. The method of claim 1, wherein the acid is selected from inorganic acids, organic acids, and combinations thereof.

8. The method of claim 1, wherein the acid is present in the mixture in an amount in the range of about 0.05 eq to about 1.5 eq, or wherein the acid is present in the mixture in an amount less than about 0.2 eq.

9. The method of claim 1, wherein the method step of forming the mixture occurs at a temperature in the range of about 0 °C to about 60 °C.

10. The method of claim 1, wherein the method step of heating the mixture increases the temperature of the mixture to a temperature in the range of about 50 °C to about 82 °C.

11. The method of claim 1, wherein the method step of adding a second amount of the compound of Formula I comprises: (A) discretely adding the second amount of the compound of Formula I, (B) continuously adding the second amount of the compound of Formula I, or (C) dropwise addition of the second amount of the compound of Formula I.

12. The method of claim 1, wherein the method step of adding a second amount of the compound of Formula I occurs over a period of time in the range of about 3 hours to about 7 hours.

13. The method of claim 1, wherein the second amount of the compound of Formula I is greater than the first amount of the compound of Formula I, such as wherein the second amount of the compound of Formula I is at least twice the first amount of the compound of Formula I.

14. The method of claim 1, wherein the second amount of the compound of Formula I is at least 70 wt% of the total amount of the compound of Formula I.

15. The method of claim 1, wherein at least one method step further comprises stirring the mixture.

## Patentansprüche

1. Verfahren zur Herstellung einer Verbindung der Formel II, wobei 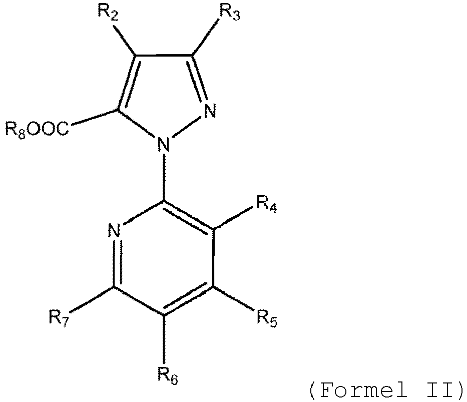
R₈ aus Wasserstoff und C₁-C₄-Alkyl ausgewählt ist und
R₂-R₇ jeweils unabhängig aus Wasserstoff, Halogen, C₁-C₄-Alkyl und halogeniertem C₁-C₄-Alkyl ausgewählt sind, wobei das Verfahren Folgendes umfasst:
I) Bilden einer Mischung, umfassend
A) eine erste Menge einer Verbindung der Formel I, wobei 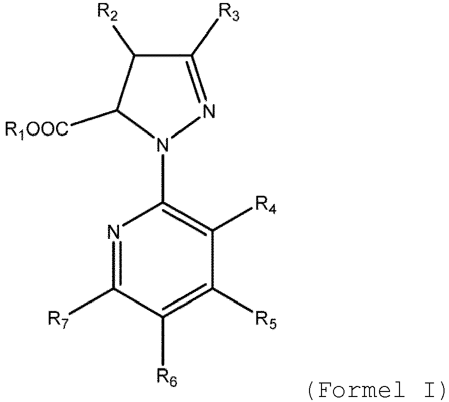
R₁ aus Wasserstoff und C₁-C₄-Alkyl ausgewählt ist und
R₂-R₇ jeweils unabhängig aus Wasserstoff, Halogen, C₁-C₄-Alkyl und halogeniertem C₁-C₄-Alkyl ausgewählt sind;
B) ein Oxidationsmittel und
C) ein organisches Lösungsmittel;
II) Erhitzen der Mischung;
III) Zugeben einer Säure zu der Mischung;
IV) Zugeben einer zweiten Menge der Verbindung der Formel I zu der Mischung und
V) Vervollständigen der Reaktion der Mischung.

2. Verfahren nach Anspruch 1, wobei das Oxidationsmittel aus Wasserstoffperoxid, organischen Peroxiden, Kaliumpersulfat, Natriumpersulfat, Ammoniumpersulfat, Kaliummonopersulfat, Natriummonopersulfat, Kaliumpermanganat und Kombinationen davon ausgewählt wird.

3. Verfahren nach Anspruch 1, wobei das Oxidationsmittel in der Mischung in einer Menge im Bereich von etwa 1,0 Äq. bis etwa 2,0 Äq. vorliegt.

4. Verfahren nach Anspruch 1, wobei das Oxidationsmittel eine D50-Teilchengröße im Bereich von etwa 10 µm bis etwa 200 µm aufweist.

5. Verfahren nach Anspruch 1, wobei das Lösungsmittel aus Ethern, Estern, aprotischen organischen Lösungsmitteln und Kombinationen davon ausgewählt wird.

6. Verfahren nach Anspruch 1, wobei die erste Menge der Verbindung der Formel I etwa 5 Gew.-% bis etwa 40 Gew.-% der Gesamtmenge der Verbindung der Formel I beträgt oder wobei die erste Menge der Verbindung der Formel I weniger als 30 Gew.-% der Gesamtmenge der Verbindung der Formel I beträgt.

7. Verfahren nach Anspruch 1, wobei die Säure aus anorganischen Säuren, organischen Säuren und Kombinationen davon ausgewählt wird.

8. Verfahren nach Anspruch 1, wobei die Säure in der Mischung in einer Menge im Bereich von etwa 0,05 Äq. bis etwa 1,5 Äq. vorliegt oder wobei die Säure in der Mischung in einer Menge von weniger als etwa 0,2 Äq. vorliegt.

9. Verfahren nach Anspruch 1, wobei der Verfahrensschritt des Bildens der Mischung bei einer Temperatur im Bereich von etwa 0 °C bis etwa 60 °C erfolgt.

10. Verfahren nach Anspruch 1, wobei im Verfahrensschritt des Erhitzens der Mischung die Temperatur der Mischung auf eine Temperatur im Bereich von etwa 50 °C bis etwa 82 °C erhöht wird.

11. Verfahren nach Anspruch 1, wobei der Verfahrensschritt des Zugebens einer zweiten Menge der Verbindung der Formel I Folgendes umfasst: (A) diskretes Zugeben der zweiten Menge der Verbindung der Formel I, (B) kontinuierliches Zugeben der zweiten Menge der Verbindung der Formel I oder (C) tropfenweises Zugeben der zweiten Menge der Verbindung der Formel I.

12. Verfahren nach Anspruch 1, wobei der Verfahrensschritt des Zugeben einer zweiten Menge der Verbindung der Formel I über einen Zeitraum im Bereich von etwa 3 Stunden bis etwa 7 Stunden erfolgt.

13. Verfahren nach Anspruch 1, wobei die zweite Menge der Verbindung der Formel I größer ist als die erste Menge der Verbindung der Formel I, wie wobei die zweite Menge der Verbindung der Formel I mindestens zweimal so groß ist wie die erste Menge der Verbindung der Formel I.

14. Verfahren nach Anspruch 1, wobei die zweite Menge der Verbindung der Formel I mindestens 70 Gew.-% der Gesamtmenge der Verbindung der Formel I beträgt.

15. Verfahren nach Anspruch 1, wobei mindestens ein Verfahrensschritt ferner das Rühren der Mischung umfasst.

## Revendications

1. Procédé de préparation d'un composé de formule II, 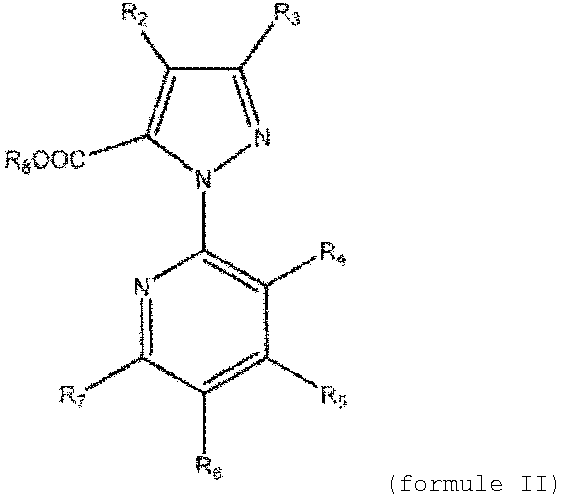
R₈ étant choisi parmi hydrogène et C₁-C₄ alkyle ; et
chacun parmi R₂ - R₇ étant indépendamment choisi parmi hydrogène, halogène, C₁-C₄ alkyle, et C₁-C₄ alkyle halogéné, le procédé comprenant
I) la formation d'un mélange comprenant
A) une première quantité d'un composé de formule I, 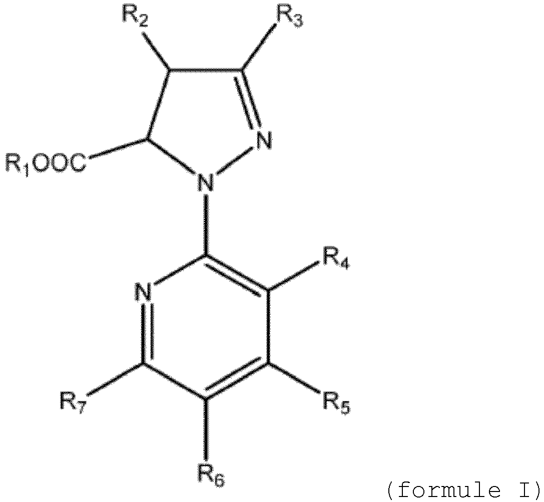
R₁ étant choisi parmi hydrogène et C₁-C₄ alkyle ; et
chacun parmi R₂ - R₇ étant indépendamment choisi parmi hydrogène, halogène, C₁-C₄ alkyle, et C₁-C₄ alkyle halogéné ;
B) un agent oxydant ; et
C) un solvant organique ;
II) le chauffage du mélange ;
III) l'ajout d'un acide au mélange ;
IV) l'ajout d'une deuxième quantité du composé de formule I au mélange ; et
V) la complétion de la réaction du mélange.

2. Procédé selon la revendication 1, l'agent oxydant étant choisi parmi le peroxyde d'hydrogène, des peroxydes organiques, le persulfate de potassium, le persulfate de sodium, le persulfate d'ammonium, le monopersulfate de potassium, le monopersulfate de sodium, le permanganate de potassium, et des combinaisons correspondantes.

3. Procédé selon la revendication 1, l'agent oxydant étant présent dans le mélange en une quantité dans la plage d'environ 1,0 éq à environ 2,0 éq.

4. Procédé selon la revendication 1, l'agent oxydant possédant une taille de particule D50 dans la plage d'environ 10 µm à environ 200 µm.

5. Procédé selon la revendication 1, le solvant étant choisi parmi des éthers, des esters, des solvants organiques aprotiques et des combinaisons correspondantes.

6. Procédé selon la revendication 1, la première quantité du composé de formule I étant d'environ 5 % en poids à environ 40 % en poids de la quantité totale du composé de formule I, ou la première quantité du composé de formule I étant moins de 30 % en poids de la quantité totale du composé de formule I.

7. Procédé selon la revendication 1, l'acide étant choisi parmi des acides organiques, des acides organiques et des combinaisons correspondantes.

8. Procédé selon la revendication 1, l'acide étant présent dans le mélange en une quantité dans la plage d'environ 0,05 éq à environ 1,5 éq, ou l'acide étant présent dans le mélange en une quantité inférieure à environ 0,2 éq.

9. Procédé selon la revendication 1, l'étape de procédé de formation du mélange ayant lieu à une température dans la plage d'environ 0 °C à environ 60 °C.

10. Procédé selon la revendication 1, l'étape de procédé de chauffage du mélange augmentant la température du mélange jusqu'à une température dans la plage d'environ 50 °C à environ 82 °C.

11. Procédé selon la revendication 1, l'étape de procédé d'ajout d'une deuxième quantité du composé de formule I comprenant : (A) l'ajout de manière discrète de la deuxième quantité du composé de formule I, (B) l'ajout de manière continue de la deuxième quantité du composé de formule I, (C) l'ajout goutte-à-goutte de la deuxième quantité du composé de formule I.

12. Procédé selon la revendication 1, l'étape de procédé d'ajout d'une deuxième quantité du composé de formule I ayant lieu sur une période de temps dans la plage d'environ 3 heures à environ 7 heures.

13. Procédé selon la revendication 1, la deuxième quantité du composé de formule I étant supérieure à la première quantité du composé de formule I, tel que la deuxième quantité du composé de formule I étant au moins le double de la première quantité du composé de formule I.

14. Procédé selon la revendication 1, la deuxième quantité du composé de formule I étant au moins 70 % en poids de la quantité totale du composé de formule I.

15. Procédé selon la revendication 1, au moins une étape de procédé comprenant en outre une agitation du mélange.
